# EUROPEAN PATENT APPLICATION

(11) **EP 2 425 722 A2**
(43) Date of publication of application: **07.03.2012**
(21) Application number: 11190478.5
(22) Date of filing: 03.04.2007
(51) Int. Cl.: A23L 1/29, A23L 1/304, A23L 1/308, A61K 31/715, A61K 31/722, A61K 31/785, A61K 31/795, A61K 33/06, A61K 33/24, A61P 3/12, A61P 13/12, A61P 19/10, A23L 2/52, A61K 33/30, A61K 45/06

(54) **Drugs and food products for hypophosphoric normo-, hyper- and hypoproteic diets, and hypophosphoric beverages**

(30) Priority: 10.04.2006 IT RM20060204
(62) Divisional of application: 07727701.0
(71) Applicant: Nielsen Structure Finance S.A., 1724 Luxembourg (LU)
(72) Inventor: Savica, Vincenzo, 98123 Messina (IT)
(74) Representative: Valenza, Silvia

(57) **Abstract**

The present invention relates to an alimentary component comprising a physiologically acceptable phosphorus binding agent, in particular polyallylamine hydrochloride, polyallylamine carbonate, lanthanum carbonate, aluminium hydroxide, magnesium acetate, magnesium carbonate, calcium carbonate, calcium acetate, calcium citrate, calcium alginate, styrene divinylbenzene ionic exchange resin, stabilized polynuclear iron hydroxide, calcium acetate plus magnesium carbonate and chitosan. Said component is useful in the nutrition of subjects in need of a low phosphorus level diet.

## Description

The present invention refers to the food industry, and in particular to dietary food products to be administered to subjects undergoing normo- hyper- and hypo- proteic diets being all hypophosphoric.

It is well known in the art, that patients with chronic renal failure and undergoing dialysis or conservative dietetic treatments, show high phosphoremic levels. Said parameters are responsible for many altered cardiovascular functions, which are linked to an increased mortality and morbidity rate and to renal failure development.

Currently, hyperphosphoremia is treated by administering binding drugs that bind to phosphorus during meals. The dose required, to obtain said effect in patients, is quite high and the pharmacologic compliance is often incorrect or it frequently changes, especially in case of multitherapy, in the presence of other pathologies.

As a consequence, the patients themselves reduce or stop the assumption of the prescribed phosphorus binding drugs.

Moreover, it is known that the limited assumption of phosphorus-containing food plays a negative nutritional role since it limits the assumption of phosphorus-containing proteins.

The number of patient suffering from renal failure, which don't need dialysis, undergoing to conservative dietetic treatment is increasing.

Said patients must take during meals phosphorus binding drugs and since they are usually subjected to multitherapy, they show low pharmacologic compliance to phosphorus binders Therefore, it is felt the need to quantitatively reduce the dosage of phosphorus binding drugs, also in terms of frequency and dosage severity and to improve the pharmacologic compliance of patients undergoing dialysis or conservative dietetic treatments and more in general subjects who need hypo-proteic and/or low phosphorus-content diets.

It has been found that the above discussed problems can be solved by the present invention, as herein disclosed, that comprises a substantial modification of the diet components so that subjects undergoing dietary restrictions, because of the hyperphosphatemia, can approach a normal diet and, contemporarily, maintain the multitherapy with improved compliance.

In the International Patent Application N. PCT/EP2005/056289, filed on 29 November 2005, claiming priority of 7 December 2004, the present inventor discloses chitosan as a phosphorus binding agent and its use in the manufacture of slow release drugs for the treatment of hyperphosphatemia during fasting.

Said use, as above described, is not associated with the assumption of food but, on the contrary, to the treatment of hyperphosphatemia in the time between meals.

### Summary of the invention

Therefore, it is an object of the present invention a dietary component comprising a physiologically acceptable phosphorus binding agent.

A further object of the present invention is the use of a physiologically acceptable phosphorus binding agent for the preparation of a dietary component for subjects in need of a low phosphorus-content diet.

Another object of the present invention is a food line comprising at least one component as previously disclosed.

The present invention, advantageously provides food products that act as phosphorus binding agent, in order to avoid the administration of drugs, if necessary, or the administration of drugs during nutrition at a dosage lower than the one used in the traditional therapy, and to avoid/reduce protein and alimentary phosphorus intake, particularly in dialysing patients where protein intake is important to lower the risk of malnutrition.

These and others objects of the present invention will be herein described in details.

### Detailed description of the invention

Alimentary component means any element that can be assumed by a subject for a nutritional or alimentary, optional or optative, purpose.

Example of alimentary components are food itself, that is capable to provide nutritional elements to a subject; food additives being, for examples, elements that are added to food in order to improve, adjust and change the organoleptic or nutritional characteristics of food or to improve storage, processing and manufacturing or any other use ordinary in this field, including, for example, dressings.

Pharmaceutically acceptable phosphorus binding agent means a substance capable to bind phosphorus, and at the same time being acceptable for the body, not harmful, not causing unsought side affects and non toxic.

Examples of said binding agent are phosphorus binding polymers, lanthanum salts, magnesium salts, calcium salts, ketoacid salts, anionic exchange resins, polysaccharides,that must be physiologically acceptable.

In particular, they can be, for example, polyallylamine hydrochloride, polyallylamine carbonate, lanthanum carbonate, aluminium hydroxide, magnesium acetate, magnesium carbonate, calcium carbonate, calcium acetate, calcium citrate, calcium alginate, styrene-divinylbenzene ionic exchange resin, stabilized polynuclear iron hydroxide, calcium acetate in combination with magnesium carbonate and chitosan.

In a preferred embodiment of the present invention, the phosphorus binding agent is a polyallylamine for example as disclosed in the patents US 5,496,545, US 5,667,775, US 6,756,363 and US 6,562,329, commercially available as Sevelamer (Renagel^{®}).

In a particularly preferred embodiment of the present invention, the phosphorus binding agent is chitosan, which is a polysaccharide commonly used in humans as a weight loss agent (see patent US 6,780,851 and cited references).

Nowadays, chitosan tablets are administered in hypo-caloric diets in order to reduce body weight, but the high dosage used often causes gastrointestinal diseases.

Advantageously, the present invention reduces the dosage to sustainable levels and avoids the above mentioned known diseases.

In a preferred embodiment of the present invention the component is a food product, in particular a farinaceous, as for example pasta, rice, flour, leavened or non-leavened products, including bread, biscuits, pizza, "focaccia", bakery, pastry, cakes, snacks, cookies, breadsticks, toasts, rusks, crackers, hypo-proteic and normo-proteic milk, hypo-proteic and normo-proteic dairy products and cheese.

With reference to chitosan, it has been found that in a hypo-proteic diet, supplying 0.6 grams of protein/Kg of body weight (0.5 g of animal origin), 0.0136 grams of phosphorus for each gram of protides, which correspond to 13.6 mg of phosphorus, which require 2.032 mg of chitosan for binding.

Thus, a hypo-proteic diet, supplying 35.8 grams of protein in total, produces 487 mg of phosphorus, that require 72.77 mg of chitosan to bind them, which should be present in food and divided in relation to phosphorus load taken with food in the different meals, in view of the fact that phosphorus intake can be quantified at each meal.

Thus, in view of the above mentioned facts, chitosan and the other binding agents, according to the general inventive concept of the present invention, become a key constituent of hypo-proteic and normo-proteic, food components that bind to the phosphorus produced by proteins. In this way the person skilled in the art can indicate to the subjects the suitable diet to be followed during the 24 hours, in view of their body weight and their kidney functionality (g of proteins/kg of body weight). The amount of chitosan required to bind phosphorus of food will vary quantitatively depending on the protein intake and the amount of phosphorus produced by them. Furthermore, chitosan and/or other phophorous binding agents used in the above mentioned doses, will be properly used, by introducing them in food, as additives or in the form of dressing.

The embodiment as additive or dressing, is in the form of single packaged powder to be sprinkled on food or to be introduced in hypo-proteic and normo-proteic milk, dairy products, cheese and in liquid or soluble supplements.

The present invention is also suitable for the treatment of known phosphorus-containing food such as sausages and cooked ham.

A proper amount of chitosan and/or other phophorous binding agents can be added to food depending on the protein/phosphorus rate.

In an embodiment of the present invention, the food product comprises chitosan in a daily dose of 72.77 mg. The skilled person would evaluate if said daily dose has to be divided in sub-doses, equal, different, or in portion of the component, for examples biscuits or other single-packed food. In another way, the present invention provides said component, for example food comprising around 0.15 mg of chitosan per mg of phosphorus contained in it.

In addition, chitosan plays a key role in binding and/or reducing food cholesterol and triglicerides absorption.

In a further embodiment of the present invention, Sevelamer, an orally administered phosphorus binding agent during meals for many years, can be used as a constituent or additive of hypo- or normo-proteic food in the following posology.

Since 1 g of Sevelamer binds around 150 mg of phosphorus and 3.24 g of Sevelamer are needed to bind the total amount of phosphorus in a diet giving 35.8 g of proteins (corresponding to four 800 mg tablets), the required amount of Sevelamer will vary with the protein content, protein intake and phosphorus production from proteins of the diet.

The application of the above mentioned concepts lead to the creation of dietetic lines comprising hypo-proteic food and normo-proteic food, wherein the above or other drug, such as polyallylamine carbonate, lanthanum carbonate, aluminium hydroxide, magnesium acetate, magnesium carbonate, calcium carbonate, calcium acetate, calcium citrate, calcium alginate, styrene divinylbenzene ionic exchange resin are inserted.

The amount of Sevelamer required for binding food phosphorus will quantitatively vary with the amount of phosphorus producing assumed proteins.

For the same reasons of the previous example regarding chitosan, for the example of Sevelamer, the person skilled in the field can prepare a component according to the present invention, comprising 6.66 mg of polyallylamine chloridrate per mg of phosphorus in said component.

In another preferred embodiment of the present invention, said component is a phosphorus containing beverage, for example Coca-Cola^{®}, Pepsi-Cola^{®} and their analogues, beer and other phosphorus containing drinks.

Coca-Cola^{®} contains relevant quantity of phosphorus and can be dangerous for patients suffering from nephropathy and undergoing dialysis.

In order to allow this kind patients to drink said beverages, a particularly preferred embodiment of the present invention provides Coca-Cola^{®}, Pepsi-Cola^{®} or their analogues containing chitosan.

Furthermore, considering the previously obtained data on chitosan and Sevelamer binding properties, the amount of chitosan and/or other phosphorus binding agents necessary to bind the phosphorus contained in other phosphorus containing beverages, milk, normo- and hyper-proteic dairy products and cheese, liquid or soluble powdery dietic supplements, can be easily calculated.

The above mentioned components can be inserted in food products, beverages, normo- and hiper- proteic milk together with vitamins or helpful substances, as for example L-carnitine, for nephrophatic patients who need a hypophosphoric diet and patients undergoing to dialysis who must limit phosphorus intake.

In the subjects who can have a diet with normal food, chitosan can effectively be used for the treatment of metabolic syndrome.

Thus, the present invention provides also the addition of vitamins, L-carnitine, fibres, acacia, garcinia hydroxycitrate, chromium, zinc, vanadium, cobalt and other elements that can be helpful for organic metabolism and positively interfere with lipid absorption, insulin tolerance and carbohydrate metabolism which are essential features for metabolic syndrome prevention.

The present invention can be used with reference to further pathologies other than renal or metabolic, as for example osteoporosis, for preventing vascular calcification. In fact, the increase of phosphoremia, even in case of non-pathologic kidney, causes development of vascular calcification and osteoporosis worsening.

Consequently, the administration of low amounts of phosphorus binding agents, in particular, chitosan and Sevelamer, alone or in combination with other phosphorus binding agents can prevent vascular calcification and atherosclerosis.

In view of the fact that Sevalamer can link lipids, it can be used, as chitosan, in hypo-caloric diets in an amount lower than usual.

In any case, the person skilled in the field will be able to determine binding agent appropriated doses considering the body dairy phosphorus intake and seric/plasmatic phosphorus levels. The same applies to lipids.

Consequently, a further object of the present invention is the use of a physiologically acceptable phosphorus binding agent for the preparation of an alimentary component, in particular, a food product or a dressing useful for a subject in need of a low phosphorus level diet.

In an embodiment of the invention, said subject has a chronic renal failure and can undergo to dialysis and to a normal diet or to a conservative hypo-proteic diet without dialysis.

In a further embodiment of the invention, said subject has osteoporosis or vascular calcification or atherosclerosis.

The present invention can also be applied to healthy subject, when a preventive effect versus damages due to hyper-phosphatemia is wished.

A further embodiment of the present invention is an alimentary line comprising at least a previously described component.

The person skilled in the art will easily carry out said alimentary line by adequately combining food products, beverages, additives and dressings comprising a phosphorus binding agent according to the present description.

The field of the invention is evident, with particular attention to food industry and more in particular to food products for subjects who have specific therapeutic or dietary needs.

The person skilled in the field of food technology can perfectly carry out the present invention by using his common general knowledge and by modifying it in view of the combination between the binding agent and the alimentary component chosen.

For example, the skilled person can determinate, from his own knowledge, how to introduce the binding agent into the component, for example when it is raw or baked or subject to a manufacturing process.

The person skilled in medicine together with a nutrition expert, can evaluate the amount of binding agent to be introduced in the food component by considering the subject conditions, the phosphorus or protides content of the component and other considerations common in the field.

The skilled in the art can also manufacture an alimentary line specifically aimed to a particular human population or for a generic use. For example, the line can comprise food as pasta, bread, breadsticks or similar, dressings, sauces, toppings, additives, as flavouring agents, in the form of bags, alcoholic or non-alcoholic beverages.

## Claims

1. Alimentary component comprising a physiologically acceptable phosphorus binding agent, wherein said binding agent is present in a proper amount depending on the protein intake and the phosphorous produced by said alimentary component; wherein said binding agent is selected from the group consisting of phosphorus binding polysaccharides, phosphorus binding polymers, lanthanum salts, magnesium salts, calcium salts, ketoacid salts, anionic exchange resins,.

2. Component according to claim 1, selected in the group consisting of food, alimentary additive and dressing.

3. Component according to anyone of claims 1 or 2, , wherein said binding agent is selected from the group consisting of chitosan, polyallylamine hydrochloride, polyallylamine carbonate, lanthanum carbonate, aluminium hydroxide, magnesium acetate, magnesium carbonate, calcium carbonate, calcium acetate, calcium citrate, calcium alginate, styrene divinylbenzene ionic exchange resin, and calcium acetate plus magnesium carbonate.

4. Component according to anyone of claims 1-3 being a farinaceous.

5. Component according to claim 4, selected from the group consisting of pasta, rice, cereals, leavened or non-leavened products.

6. Component according to claim 5 selected from the group consisting of bread, biscuits, pizza, bakery, pastry, cakes, snacks, cookies, breadsticks, toasts, rusks, crackers and derivatives.

7. Component according to anyone of claims 1-3 chosen from the group comprising normo-proteic milk, dairy products, cheese, sausages, cooked ham, liquid or soluble alimentary additives.

8. Component according to anyone of the preceding claims comprising an amount of chitosan equal to a daily dose of about 72.77 mg.

9. Component according to anyone of the preceding claims comprising 0.15 mg of chitosan per mg of phosphorus in said component.

10. Component according to anyone of claims 1-6 comprising about 6.66 mg of polyallylamine hydrochloride per mg of phosphorus in said component.

11. Component according to anyone of claims 1-3 in the form of beverage.

12. Component according to claim 12 selected from the group consisting of beer and other phosphorus containing drinks.

13. Component according to anyone of claims 1-13, further comprising L-carnitine, fibres, acacia, garcinia hydroxycitrate, chromium, zinc, vanadium, cobalt.

14. Use of a physiologically acceptable phosphorus binding agent for the preparation of a component of anyone of claims 1-13 useful in the nutrition of subjects in need of a low phosphorus level diet.

15. Use according to claim 14 wherein said binding agent is selected from the group consisting of a phosporus binding polysaccharides, a phosphorus binding polymer, a lanthanum salt, a magnesium salt, a calcium salt, a ketoacid salt, an anionic exchange resin.

16. Use according to claim 15 wherein said binding agent is selected from the group consisting of chitosan, polyallylamine hydrochloride, polyallylamine carbonate, lanthanum carbonate, aluminium hydroxide, magnesium acetate, magnesium carbonate, calcium carbonate, calcium acetate, calcium citrate, calcium alginate, styrene divinylbenzene ionic exchange resin, calcium acetate plus magnesium carbonate.

17. Use according anyone of claims 14-16 wherein said subject suffers from chronic renal failure in conservative treatment.

18. Use according to claim 17 wherein said subject undergoes dialysis and a normal diet.

19. Use according to claim 18 wherein said subject undergoes a conservative diet.

20. Use according anyone of claims 14-16, wherein said subject suffers from osteoporosis.

21. Use according anyone of claims 14-17 or 20 wherein said subject suffers from vascular calcification.

22. Alimentary line comprising at least one component of anyone of claims 1-13.
